Europäisches Patentamt

European Patent Office

Office européen des brevets

(11) Veröffentlichungsnummer: **0 251 046 B1**

# EUROPÄISCHE PATENTSCHRIFT

(45) Veröffentlichungstag der Patentschrift: 26.06.91

(51) Int. Cl.5: **A61K 49/02**

(21) Anmeldenummer: 87108753.2

(22) Anmeldetag: **19.06.87**

(54) **Diagnostikum zur szintigraphischen Darstellung von bösartigen Tumoren.**

(30) Priorität: 27.06.86 DE 3621570

(43) Veröffentlichungstag der Anmeldung:
07.01.88 Patentblatt 88/01

(45) Bekanntmachung des Hinweises auf die
Patenterteilung:
26.06.91 Patentblatt 91/26

(84) Benannte Vertragsstaaten:
AT BE CH DE ES FR GB GR IT LI LU NL SE

(56) Entgegenhaltungen:
EP-A- 142 641
EP-A- 199 085
FR-A- 2 309 215

Nuklearmedizin, Band 25, 1986, Seiten 28-30
C.Schümichen et al.: Tu mor localization and
biodistribution of 99m Tc-
muramylpolypeptide (MPP)in the rat

CHEMICAL ABSTRACTS, Band 55, Nr. 17, 21.
August 1961, Spalte 16674 f-g, Columbus,
Ohio, US; J.PRIMOSIGH et al.: Chemical characterization of mucopeptides released from
the Escherichia coli B cell wall by enzymetic
action & BIOCHIM. ET BIOPHYS. ACTA 46,
68-80(1961)

(73) Patentinhaber: HOECHST AKTIENGESELL-
SCHAFT
Postfach 80 03 20
W-6230 Frankfurt am Main 80(DE)

(72) Erfinder: Kuhlmann, Ludwig. Dr.
Gartenstrasse 17
W-6238 Hofheim am Taunus(DE)
Erfinder: Seibert, Gerhard, Prof. Dr.
Gläserweg 21
W-6100 Darmstadt(DE)
Erfinder: Steinsträsser, Axel, Dr. Dr.
Zum Morgengraben 26
W-6237 Liederbach(DE)
Erfinder: Bremer, Karl-Heinz, Dr.
Am Rehsteig 19
W-6232 Bad Soden am Taunus(DE)
Erfinder: Die anderen Erfinder haben auf ihre
Nennung verzichtet

EP 0 251 046 B1

**Beschreibung**

Die Erfindung betrifft ein Diagnostikum zur szintigraphischen Darstellung von bösartigen Tumoren, ein Verfahren zur szintigraphischen Darstellung von bösartigen Tumoren mit diesem Diagnostikum und ein Herstellungsverfahren für das Diagnostikum.

Schon lange besteht der Wunsch, ein Diagnostikum zur Verfügung zu haben, das die Größe und Lage bösartiger Tumore schon frühzeitig erkennen läßt. Es hat nicht an Versuchen gefehlt, dieses Ziel mit Hilfe nuklearmedizinischer Methoden zu erreichen, die das Verteilungsmuster von mit $\gamma$-strahlenden Nukliden gekoppelten Substanzen im menschlichen Körper empfindlich nachweisen und bildlich darstellen können. Der Erfolg dieser Methoden hängt davon ab, ob es gelingt, Stoffe zu finden, die sich spezifisch oder unspezifisch in bösartigen Tumoren anreichern.

Zur Tumorszintigraphie ist schon eine kaum noch zu übersehende Anzahl von Tumor-assoziierten Substanzen eingesetzt worden, die jedoch nicht Tumor-spezifisch sind und deren Tumoraffinität häufig unbekannt ist. Hierzu gehören anorganische Substrate wie Gallium-67-zitrat, Indium-111-chlorid und Wismut-206-chlorid. Aber auch Metabolite des Tumorstoffwechsels wie Stickstoff-13-glutamat und Selen-75-methionin wurden schon für den szintigraphischen Nachweis von Tumoren eingesetzt (vgl. E. Henze, "Szintigraphische Lokalisationsdiagnostik von Tumoren", Münch. med. Wschr. 127, 182 - 184 (1985)). Schließlich sind auch schon radioaktiv markierte Cytostatika wie Cobalt-57-bleomycin oder mit Tumoren assoziierte Proteine wie Jod-131-antifibrin eingesetzt worden. Allen diesen Substanzen ist gemeinsam, daß sie sich häufig nur in geringen Konzentrationen im Tumor anreichern, so daß eine eindeutige Bestimmung der Lage des Tumors nicht möglich ist, insbesondere dann nicht, wenn es sich um kleine Tumorherde handelt.

Die Tumorszintigraphie hat in den letzten Jahren jedoch auch durch die Verfügbarkeit radioaktiv markierter, tumorspezifischer monoklonaler Antikörper oder deren immunaktiver Fragmente wesentliche Fortschritte gemacht (vgl. A.M. Keenan, J.C. Harbert and S.M. Larson, "Monoclonal Antibodies in Nuclear Medicine", The Journal of Nuclear Medicine, Volume 26, 531 - 537 (1985)). Eine verbesserte in vivo-Kinetik dieser Antikörper hat bereits sehr ermutigende Resultate gebracht. Die verfügbare nuklearmedizinische Technik erlaubt eine quantitative und tomographische Darstellung. Größere klinische Erfahrungen hinsichtlich Sensitivität, Spezifität oder Nebenwirkungen liegen jedoch wegen der Heterogenität der bisher verwendeten Antikörper noch nicht vor. Hinzu kommt, daß monoklonale Antikörper nur die Tumore erfassen können, die das genau entsprechende Antigen auf der Zelloberfläche tragen. Die hohe Spezifität der monoklonalen Antikörper erlaubt deshalb keinen generellen Nachweis von beliebigen Tumoren.

Aus der Europäischen Patentanmeldung 142 641 ist nun auch ein Mittel für die Diagnose und Therapie von Tumoren bekannt geworden, welches einen mit einem radioaktiven Strahler, einem Farbstoff oder einem Cytostatikum markierten Immunmodulator enthält. Dieses Mittel wird vorzugsweise zusammen mit einem Cocktail bestehend aus einem Aldehyd und einem Alkohol, verabreicht. Unter den bevorzugten Immunmodulatoren werden Muraminsäuredipeptid-Derivate, Peptidoglykane oder peptidoglykanfreie Extrakte aus bestimmten Bakterienarten verstanden. Bei Patienten, die 30 Minuten vor der Gabe des Diagnostikums den genannten Cocktail getrunken hatten, war ein Tumornachweis möglich.

Es wurde nun gefunden, daß man auf die Verabreichung eines aus einem Aldehyd und einem Alkohol bestehenden Cocktail verzichten kann, wenn man zur szintigraphischen Darstellung von bösartigen Tumoren ein Diagnostikum einsetzt, das eine bei der enzymatischen Aufarbeitung von Murein aus Escherichia coli gewonnene Substanz enthält, an die mit Hilfe eines Komplexbildners ein Radionuklid gebunden ist.

Bei der durch die enzymatische Aufarbeitung von Murein gewonnene Substanz handelt es sich um ein Gemisch aus Komponenten wie sie z.B. beschrieben sind von B. Glauner und U. Schwarz, in "The Analysis of Murein with HPLC", in The Target of Penicillin, Seite 29 - 34, Berlin 1983.

Hinzu kommt ein Anteil cyclischer Muropeptide, z.B. Muropeptid $C_4$, wie beschrieben bei W. Weidel und H. Pelzer, Adv. in Enzymol.26, 193 - 232 (1964) und ein Anteil höhermolekularer Mureinfragmente vom Molekulargewicht bis etwa 10000.

Diese Substanz wird gewonnen, indem man nach den literaturbekannten Verfahren hergestelltes Murein aus E. coli-Zellen (10 mg/ml) in 0,05 M Ammoniumacetat suspendiert und mit 10 $\mu$g/ml Hühnereiweiß-Lysozym 18 Stunden bei 37°C inkubiert. Nach Sedimentation unlöslicher Bestandteile bei 50000 x g wird mit Chloroform extrahiert und die wäßrige Phase getrocknet. Ammoniumacetat wird bei 50°C im Vakuum über 65 Stunden entfernt.

An die freien Aminogruppen dieser Substanz wird ein Komplexbildner wie Ethylendiamintetraessigsäure, Diethylentriaminpentaessigsäure oder Nitrilotriessigsäure gebunden, der ein Radionuklid trägt. Vorzugsweise werden hierfür Technetium-99m oder Indium-111 eingesetzt.

Es ist ein besonderer Vorteil, daß das erfindungsgemäße Diagnostikum durch die Aufarbeitung der

2

Zellwände aus Escherichia coli gewonnen wird, da dieser Mikroorganismus als eingefrorene Zellmasse käuflich erhältlich ist. Die durch die enzymatische Aufarbeitung von Murein gewonnene Substanz läßt man zweckmäßigerweise bei Raumtemperatur mit dem Komplexbildner reagieren. Nach Abtrennung des überschüssigen Komplexbildners wird die Lösung lyophilisiert und mit der gewünschten Aktivität des Radionuklids versetzt. Anschließend erfolgt eine Sterilfiltration und die Einstellung mit isotonischer Kochsalzlösung auf die gewünschte Aktivitätskonzentration.

Das so gewonnene Diagnostikum ist überraschenderweise geeignet, ganz unterschiedliche bösartige Tumore sichtbar zu machen. Sowohl Weichteiltumore als auch Knochentumore können gut erkannt werden.

Die Herstellung und die Anwendung des Diagnostikums werden durch die folgenden Beispiele erläutert:

Beispiel 1

Ankoppeln von Diethylentriaminpentaessigsäure (DTPA)

10 mg nach allgemein bekannten Verfahren enzymatisch aufgearbeitetes Murein aus E. coli wird in 0,5 ml 0,05 m Bicarbonatpuffer pH 8,2 gelöst, bei Raumtemperatur mit 10 mg bicyclisches DTPA-Anhydrid versetzt und 10 min bei Raumtemperatur stehengelassen. Das überschüssige DTPA wird chromatographisch (Trägermaterial: Kieselgel RP-18; Fließmittel: 0,1 m Phosphatlösung pH 4,69 und Methanol : Wasser 70 : 30) abgetrennt. Die erhaltene Präparatelösung wird in Portionen vereinzelt und lyophilisiert.

Beispiel 2

Umsetzung mit Indium-111

Das Lyophilisat wird in 0,1 m Ammoniumcitratpuffer pH = 6,5 gelöst und mit der gewünschten Aktivität, z.B. 100 MBq, an Indium-111-citrat bei Raumtemperatur vermischt. Das Indium-111-citrat wird aus dem kommerziell erhältlichen Indium-111-chlorid durch 1 : 1 Verdünnung mit 0,1 m Ammonium-citrat pH = 6,5 hergestellt. Nach 10 min Stehen bei Raumtemperatur wird die Lösung durch einen Filter sterilfiltriert und mit isotonischer Kochsalzlösung auf die gewünschte Aktivitätskonzentration eingestellt.

Die radiochemische Reinheit kann durch Hochleistungs-Flüssigkeitschromatrographie geprüft werden.

**Trägermaterial: Kieselgel RP-18**

**Fließmittel:**  20 min  $0,1m\ H_3PO_4$ pH= 4,69

120 min 0-100 % $0,1m\ H_3PO_4$ pH= 4,69+15 % $CH_3OH$

Das Präparat ist nach Filtration durch den Sterilfilter injektionsfertig. Die Markierungsprozedur kann sowohl beim Hersteller als auch Anwender durchgeführt werden.

Beispiel 3

Tierversuch

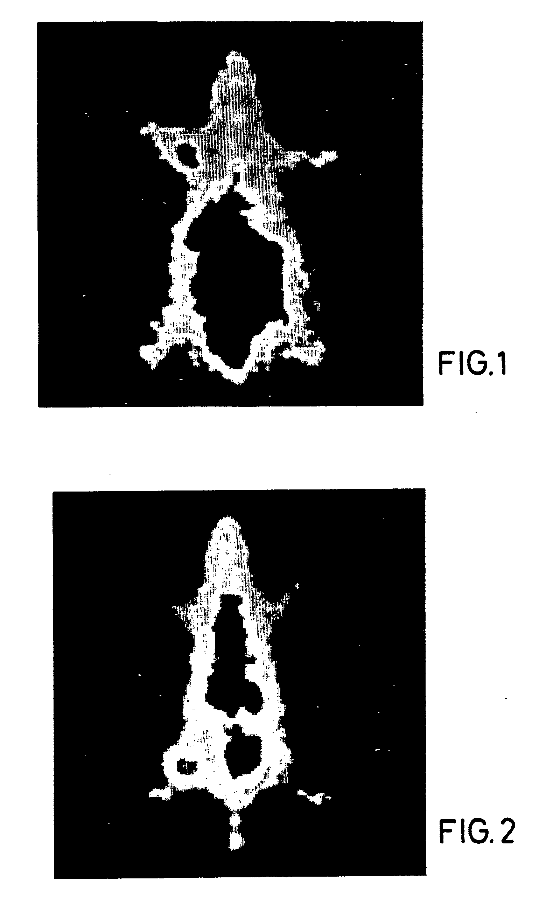

In die Muskulatur des rechten Vorderbeins einer Ratte (Sprague-Dawley) wurde ein Walker-Carcinom 256 (Weichteiltumor) implantiert. Nachdem sichergestellt war, daß sich der Tumor normal entwickelte, wurden dem Tier 0,5 ml (0,3 MBq Indium-111) des Präparates intravenös injiziert. Das nach 2 h erhaltene Szintigramm ist in Fig. 1 wiedergegeben. Der Tumor ist am rechten Vorderbein deutlich abgrenzbar. Der Tumor/Muskel-Quotient betrug 4.

Beispiel 4

Tierversuch

Einer Ratte (Sprague-Dawley), der am rechten Hinterbein ein Osteosarkom (Knochentumor) implantiert worden war, wurden 0,5 ml (0,3 MBq Indium-111) des Präparates i.v. appliziert. Im Szintigramm nach 2 h (Fig. 2), ist der Tumor eindeutig zu lokalisieren. Das gemessene Tumor/Muskel-Verhältnis war ebenfalls 4.

**Ansprüche**

Patentansprüche für folgende Vertragsstaaten : BE, CH, DE, FR, GB, IT, LI, LU, NL, SE

1. Diagnostikum zur szintigraphischen Darstellung von bösartigen Tumoren, dadurch gekennzeichnet, daß es ein Radionuklid enthält, das mit Hilfe eines Komplexbildners an die durch die enzymatische Aufarbeitung von Murein aus Escherichia coli gewonnene Substanz gebunden ist.

2. Diagnostikum nach Anspruch 1, dadurch gekennzeichnet, daß als Radionuklid Technetium-99m oder Indium-111 eingesetzt werden.

3. Diagnostikum nach Anspruch 1, dadurch gekennzeichnet, daß als Komplexbildner Diethylentriaminpentaessigsäure eingesetzt wird.

4. Verfahren zur Herstellung des Diagnostikums nach Anspruch 1, dadurch gekennzeichnet, daß man die durch die Aufarbeitung des Mureins gewonnene Substanz nach Bindung an dem Komplexbilder lyophilisiert und erst zur Markierung wieder in Lösung bringt.

Patentansprüche für folgende Vertragsstaaten : AT, ES, GR.

1. Verfahren zur Herstellung eines Diagnostikums zur szintigraphischen Darstellung von bösartigen Tumoren, dadurch gekennzeichnet, daß ein Radionuklid mit Hilfe eines Komplexbildners an eine durch die enzymatische Aufarbeitung von Murein aus Escherichia coli gewonnene Substanz gebunden wird.

2. Verfahren nach Anspruch 1, dadurch gekennzeichnet, daß man die durch die Aufarbeitung des Mureins gewonnene Substanz nach Bindung an den Komplexbildner lyophilisiert und erst zur Markierung wieder in Lösung bringt.

3. Verfahren nach Anspruch 1 oder 2, dadurch gekennzeichnet, daß als Radionuklid Technetium-99m oder Indium-111 eingesetzt wird.

4. Verfahren nach Anspruch 1 oder 2, dadurch gekennzeichnet, daß als Komplexbildner Diethylentriaminpentaessigsäure eingesetzt wird.

**Claims**

Claims for the following Contracting States : BE, CH, DE, FR, GB, IT, LI, LU, NL, SE

1. A diagnostic aid for the scintigraphic visualization of malignant tumors, which contains a radionuclide which has been bonded by means of a complex-forming agent to the substance obtained by the enzymatic processing of murein from Escherichia coli.

2. A diagnostic aid as claimed in claim 1, wherein technetium-99m or indium-111 are used as radionuclide.

3. A diagnostic aid as claimed in claim 1, wherein diethylenetriaminepentaacetic acid is used as complex-forming agent.

4. A process for the preparation of the diagnostic aid as claimed in claim 1, which comprises the substance which has been obtained by the processing of murein and bonded to the complex-forming agent being freeze-dried and not redissolved until labeling is carried out.

Claims for the following Contracting States : AT, ES, GR

1. A process for the preparation of a diagnostic aid for the scintigraphic visualization of malignant tumors, which comprises bonding a radionuclide by means of a complex-forming agent to a substance obtained by the enzymatic processing of murein from Escherichia coli.

2. The process as claimed in claim 1, wherein the substance which has been obtained by the processing of murein and bonded to the complex-forming agent is freeze-dried and not redissolved until labeling is

EP 0 251 046 B1

carried out.

3. The process as claimed in claim 1 or 2, wherein technetium-99m or indium-111 is used as radionuclide.

4. The process as claimed in claim 1 or 2, wherein diethylenetriaminepentaacetic acid is used as complex-forming agent.

**Revendications**

Revendications pour les Etats contractants suivants : BE, CH, DE, FR, GB, IT, LI, LU, NL, SE

1. Agent de diagnostic pour la représentation par scintigraphie de tumeurs malignes, caractérisé en ce qu'il contient un radionuclide qui est lié à l'aide d'un complexant à la substance obtenue par le traitement enzymatique de muréine provenant de <u>Escherichia</u> <u>coli</u>.

2. Agent de diagnostic selon la revendication 1, caractérisé en ce que l'on utilise en tant que radionuclide le technétium-99m ou l'indium-111.

3. Agent de diagnostic selon la revendication 1, caractérisé en ce que l'on utilise en tant que complexant l'acide diéthylènetriaminepentaacétique.

4. Procédé pour la préparation de l'agent de diagnostic selon la revendication 1, caractérisé en ce qu'on lyophilise la substance obtenue par le traitement de la muréine, après liaison au complexant, et on ne la remet en solution que pour le marquage.

Revendications pour les Etats contractants suivants : AT, ES, GR

1. Procédé pour la préparation d'un agent de diagnostic pour la représentation par scintigraphie de tumeurs malignes, caractérisé en ce qu'on lie un radionuclide, a l'aide d'un complexant, à une substance obtenue par le traitement enzymatique de muréine provenant de <u>Escherichia</u> <u>coli</u>.

2. Procédé selon la revendication 1, caractérisé en ce qu'on lyophilise la substance obtenue par le traitement de la muréine, après liaison au complexant, et on ne la remet en solution que pour le marquage.

3. Procédé selon la revendication 1 ou 2, caractérisé en ce que l'on utilise en tant que radionuclide le technétium-99m ou l'indium-111.

4. Procédé selon la revendication 1 ou 2, caractérisé en ce que l'on utilise en tant que complexant l'acide diéthylènetriaminepentaacétique.

FIG.1

FIG.2